Europäisches Patentamt

European Patent Office

Office européen des brevets

(19)

(11) Veröffentlichungsnummer: **0 087 611**
**B1**

(12) # EUROPÄISCHE PATENTSCHRIFT

(45) Veröffentlichungstag der Patentschrift:
**27.11.85**

(51) Int. Cl.⁴: **G 01 N 33/36**

(21) Anmeldenummer: **83101070.7**

(22) Anmeldetag: **04.02.83**

(54) Vorrichtung zur Bestimmung der Substanzmenge oder der Dichte von Faserverbänden, insbesondere des Substanzquerschnittes von Faserbändern.

(30) Priorität: **19.02.82 CH 1040/82**

(43) Veröffentlichungstag der Anmeldung:
**07.09.83 Patentblatt 83/36**

(45) Bekanntmachung des Hinweises auf die Patenterteilung:
**27.11.85 Patentblatt 85/48**

(84) Benannte Vertragsstaaten:
**DE FR GB IT**

(56) Entgegenhaltungen:
**CH - A - 553 981**
**DD - A - 151 225**
**GB - A - 952 886**

(73) Patentinhaber: **ZELLWEGER USTER AG, Wilstrasse 11, CH-8610 Uster (CH)**

(72) Erfinder: **Murbach, Erwin, Werrikonerstrasse, CH-8606 Nänikon (CH)**
Erfinder: **Peter, Jean, Lindhof, CH-Mönchaltorf (CH)**

(74) Vertreter: **Dipl.-Phys.Dr. Manitz Dipl.-Ing. Finsterwald Dipl.-Ing. Grämkow Dipl.-Chem.Dr. Heyn Dipl.-Phys.Rotermund, B.Sc. Morgan Robert-Koch-Strasse 1, D-8000 München 22 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

In der Textilindustrie ist die Bestimmung der Substanzmenge von Faserverbänden, insbesondere des Substanzquerschnittes von Faserbändern sehr wichtig. Dem Substanzquerschnitt von Faserbändern kommt in der Weiterverarbeitung erhebliche Bedeutung zu, da dieser die Feinheit der Garne und deren Querschnittsgleichmässigkeit direkt beeinflusst.

Primär handelt es sich darum, die Substanzmenge eines Faserverbandes zu bestimmen, ohne Rücksicht darauf, ob dieser sich in ruhendem oder in bewegtem Zustand befindet. Die Messung von laufenden Faserbändern unterscheidet sich im Prinzip nicht von der Messung unbewegter Faserverbände, lediglich die Organe zur Führung und Begrenzung der Faserbänder sind entsprechend auszubilden.

Die Substanzbestimmung durch Wägung ist insbesondere an laufenden Faserbändern praktisch unmöglich. Es sind deshalb schon zahlreiche Lösungsvorschläge aufgezeigt worden, um diesen Substanzquerschnitt indirekt zuverlässig zu messen. Als Beispiele seien genannt:

— Die rein mechanische Messung mittels Nutenwalze und Tastrolle, bei der das Faserband durch die Nutenwalze geführt und der Achsabstand zwischen Nutenwalze und Tastrolle als Mass für den Substanzquerschnitt ausgewertet wird. Bei dieser Anordnung ist der hohe mechanische Aufwand, die begrenzte Abtastfrequenz infolge grosser mechanisch zu bewegender Massen und die starke Faserkompression nachteilig.

. — Optische Messorgane sind ebenfalls bekannt, die das Absorptions- oder Reflexionsvermögen der Faserbänder ausnützen. Auch diese Messsysteme weisen einen hohen Aufwand an elektrischen und optischen Mitteln auf; ausserdem ergibt die Abhängigkeit der Lichtabsorption oder -reflexion von der Farbe des Fasermaterials unzuverlässige Resultate.

— Akustische Messorgane sind ebenfalls bekannt. Bei diesen wird die Dämpfung von Luftschallwellen, die ein Faserband durchdringen, gemessen. Die Genauigkeit vermochte aber bisher nicht zu befriedigen.

— Als weiterer Vertreter in der Reihe der Messorgane für den Substanzquerschnitt von Faserbändern sei noch das sogenannte aktiv-pneumatische Messorgan genannt. Dieses besteht im wesentlichen aus einem Trichter, an den seitlich eine Druckmessvorrichtung angeschlossen ist. Beim Durchlauf des Faserbandes durch den Trichter treten an diesem seitlichen Anschluss Druckschwankungen auf, die dem jeweiligen Querschnitt entsprechen und in geeigneten Wandlern in äquivalente Signale umgeformt werden. Dieses aktiv-pneumatische Messorgan ist an sich einfach im Aufbau; jedoch weist es als Nachteile eine Abhängigkeit des Messwertes sowohl von der Faserfeinheit (Micronairwert), als auch von der Durchlaufgeschwindigkeit auf.

Es sind auch bereits Versuche angestellt worden, bei denen die Elastizität eines einen Kanal mit definierten Abmessungen durchlaufenden Faserbandes bestimmt wird; das Faserband wird dabei als eine Feder betrachtet, welche zusammen mit der Masse des Faserbandes ein mechanisches Schwingungssystem bildet. Nachteilig ist dabei der hohe technische Aufwand für Geber, Empfänger und deren Zubehör für die Bestimmung der Substanzmenge des Fasermaterials.

Aus der DD-A Nr. 151225 ist eine Vorrichtung zum Messen der linearen Dichte oder der Massenungleichheit von textilindustriellen Halberzeugnissen, insbesondere von Fadenbändern bekannt, wobei ein Fadenband einen Kanal durchläuft, in den seitlich durch eine Öffnung Luft geblasen wird. Änderungen der linearen Dichte oder der Masse des Fadenbandes bewirken eine Druckänderung in der Luftzuführung zur seitlichen Öffnung. Diese Druckänderungen werden als Messgrössen erfasst.

Die Aufgabe, die der Erfindung zugrunde liegt, besteht mithin darin, einen geringen technischen Aufwand für Geber, Empfänger und deren Zubehör zu erzielen, sowie den Wartungsaufwand für eine erfindungsgemässe Vorrichtung gering zu halten.

Die vorliegende Erfindung löst diese Aufgabe und betrifft eine Vorrichtung zur Bestimmung der Substanzmenge oder der Dichte von Faserverbänden mittels eines bezüglich Volumen definierten Raumes mit Fasern oder einem bezüglich Fläche definierten Querschnitt mit Fasern, die dadurch gekennzeichnet ist, dass in dem das Volumen bzw. den Querschnitt definierenden Kanal (6) eine Öffnung (9) vorgesehen ist, in der ein passiver Körper beweglich derart gelagert ist, dass er teilweise in den Kanal (6) hineinragt, welcher Körper durch eine Kraft entgegen der Elastizität der Fasern belastet ist, und dass Mittel zur Messung der Bewegung des passiven Körpers vorgesehen sind.

Anhand der Beschreibung und der Figuren wird das Messprinzip sowie Ausführungsbeispiele näher erläutert. Dabei zeigt

Fig. 1 schematisch das Grundprinzip des Messvorgangs,

Fig. 2 ein Kraft-Dehnungs-Diagramm,

Fig. 3 ein Messystem mit kubischem Messkörper,

Fig. 4 dasselbe System mit sphärischem Messkörper,

Fig. 5 ein praktisch ausgeführtes Messorgan gemäss Fig. 4 im Längsschnitt,

Fig. 6 dasselbe Messorgan im Querschnitt

Fig. 7 als Detail einen vergrösserten Ausschnitt auf Fig. 6 im Längsschnitt,

Fig. 8 denselben Ausschnitt von unten gesehen.

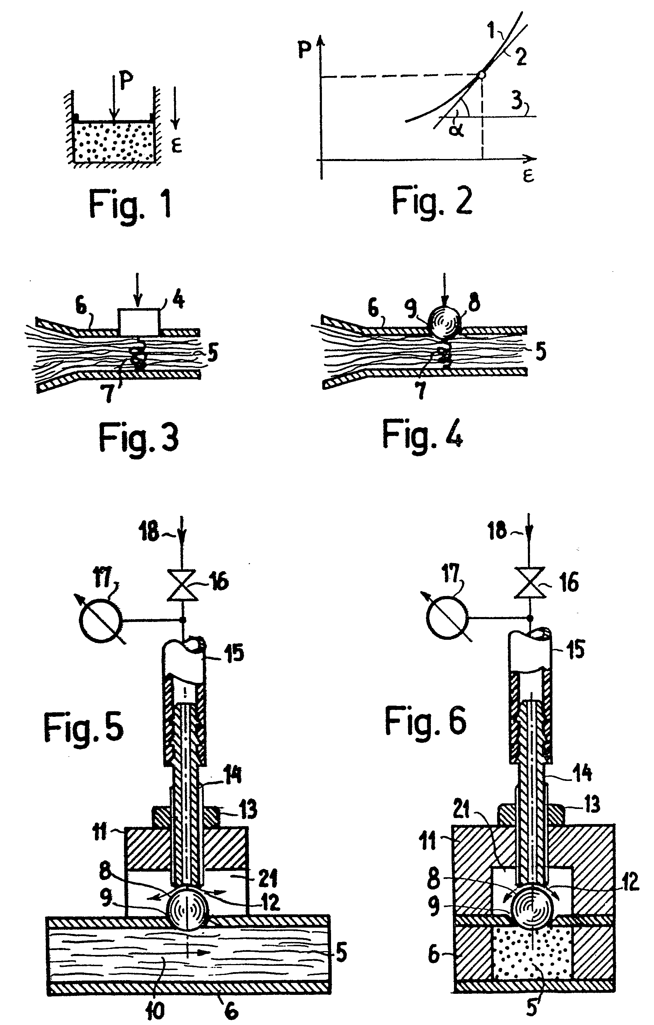

Der Erfindung liegt vorerst folgende Beobachtung zugrunde: Textile Faserverbände sind in der Regel ein Gebilde aus Fasern mit sehr grossen Luftzwischenräumen. Bei mässiger Pressung ist der reine Materialanteil (Substanzmenge) der Fasern immer noch relativ klein. So beträgt er beispielsweise an den Trichtern von Karden nur 10-20%. Wenn nun eine lockere Fasermenge gepresst wird (Fig. 1), ergibt sich ein bestimmter Verlauf der Kraft-Dehnungskurve (Fig. 2). Da an die Fasermenge keine Zugkräfte, sondern nur Druckkräfte (Pressungen) angelegt werden können, entspricht der Dehnung eine Pressung; dies zum Unterschied zu den allgemein üblichen Kraft-Dehnung-Diagrammen fester Körper. Gemäss der graphischen Darstellung in Fig. 2 ist in Abszissenrichtung die Dehnung ε (Pressung), und die Kraft P in Ordinatenrichtung als Kurvenzug 1 gezeigt. Der Elastizitätsmodul E kann in bekannter Weise in jedem Punkt durch den Tangens des Winkels α, den eine an die Kurve 1 gelegte Tangente 2 mit der Horizontalen 3 einschliesst, ausgedrückt werden:

$$E = tg\ \alpha \qquad (I)$$

Beobachtungen haben ergeben, dass einer bestimmten Dichte der Fasermenge eine bestimmte Kraft und damit ein bestimmter Elastizitätsmodul entspricht. Unter der Dichte der Fasermenge ist hierbei der Mittelwert aus dem Gemisch von Fasermaterial und der Luft in den Zwischenräumen des Faserverbandes zu verstehen. Daher gilt: Der reine Substanzquerschnitt $Q_o$ des Faserbandes ist gleich dem Querschnitt Q des das Faserband enthaltenden Kanals x Füllfaktor F.

Die Veränderung der Kraft bzw. des Elastizitätsmoduls in Funktion der Dichte ist sehr ausgeprägt. Hieraus entspringt als erster erfinderischer Gedanke der, dass an irgend einer Stelle, an welcher die gesamte Fasermenge einen definierten Raum oder Querschnitt aufweist, die Presskraft bestimmt und daraus auf die Dichte geschlossen wird. Aus der Dichte und dem genannten Raum oder Querschnitt kann hernach die reine Materialmenge bestimmt werden.

Es ist allerdings darauf hinzuweisen, dass die Kraft bzw. der Elastizitätsmodul in Funktion der Pressung bei mehreren Messvorgängen der gleichen Fasermenge in der Regel ungleiche Resultate ergibt. Vor allem bei der ersten Pressung der losen Fasermenge ist ein höherer Kraftbedarf notwendig als bei den nächstfolgenden. Für die erfindungsgemässe Bestimmung der Substanzmengen in Faserverbänden ist es daher vorteilhaft, nur die Resultate der ersten Pressung oder nur die Resultate wiederholter Pressungen auszuwerten. Besonders vorteilhaft ist es dabei, die Bestimmung der Pressung in Rohren oder im Bereich der Trichter (Trompeten) von Karden, Strecken oder dergleichen vorzunehmen, weil dort Faserverbände betriebsmässig und in der Regel kontinuierlich von einem losen Zustand in eine Verengung geführt werden und somit eigentlich immer die erste Pressung stattfindet. Ein weiterer Vorteil solcher Messstellen ist darin zu sehen, dass der äussere Faserbandquerschnitt genau definiert werden kann. Nachdem dieser Anwendungsfall wohl dem häufigsten Einsatz eines Messorgans nach der Erfindung entspricht, sollen in den nächsten Abschnitten vorwiegend Messanordnungen zur Bestimmung der Substanzmenge von Faserverbänden behandelt werden, die durch einen bestimmten Querschnitt geführt werden. Grundsätzlich sind die Überlegungen aber auch gültig für Messungen an ruhendem Material in einem genau definierten Raum.

Die Funktion der erfindungsgemässen Messvorrichtung lässt sich anhand von Fig. 3 wie folgt erklären: Der Messkörper 4, der seitlich in dem das Fasermaterial 5 enthaltenden Kanal 6 eingesetzt ist, drückt auf das Fasermaterial 5, das infolge seiner elastischen Eigenschaften als Feder 7 bezeichnet werden kann, die dem Messkörper entgegenwirkt.

Eine Weiterbildung dieses Prinzips zeigt Fig. 4. Darin besteht der Messkörper aus der in das Fasermaterial 5 eintauchenden Kalotte einer Kugel 8, die durch eine Öffnung 9 in der Kanalwand am Fasermaterial anliegt und dadurch den Kanalquerschnitt leicht verengt. Dadurch kommt die Elastizität des Fasermaterials 5 zur Wirkung, indem die Kugel 8 nach aussen gedrückt wird. Diesem Nachgeben der Kugel 8 wird nun erfindungsgemäss eine auf die Kugel 8 wirkende Kraft P entgegengerichtet. Diese Kraft wird vorzugsweise so geregelt, dass sich unabhängig von der Fasermenge im Kanalquerschnitt eine definierte Gleichgewichtslage der Kugel 8 einstellt. Die sich einstellende Kraft ist dann ein Mass für die Fasermenge im Kanalquerschnitt.

Vorzugsweise wird die Kraft P auf die Kugel 8 pneumatisch erzeugt. Es können jedoch auch andere Arten der Krafteinwirkung auf die Kugel 8, wie beispielsweise elektrische oder magnetische Mittel, verwendet werden. In jedem Fall soll bei definierter Gleichgewichtslage die resultierende Kraft bzw. bei definierter Kraft die resultierende Gleichgewichtslage der Kugel 8 in Querrichtung zur Kanalachse als charakteristischer Parameter für die Fasermenge bestimmt werden können.

Fig. 5 und 6 zeigen ein Ausführungsbeispiel einer solchen Messvorrichtung mit pneumatischer Krafterzeugung in Längs- und Querschnitt. Man erkennt den Kanal 6, durch den das Fasermaterial 5 in Richtung des Pfeiles 10 läuft. In die ringförmige, kegelige Öffnung 9 ist die Kugel 8 eingelegt.

In ein auf dem Kanal 6 fest aufgesetztes Joch 11 ist ein Düsenrohr 14 eingeschraubt und mittels einer Feststellmutter 13 fixiert. Zwischen der Kugel 8 und dem Austritt der Düse 12 besteht somit soviel Spiel, dass sich ein ringförmiger Luftspalt bilden kann, durch den die durch die Düse 12 zugeführte Druckluft entweicht. Diese Druckluft wird einer Druckluftquelle 18 entnommen. Mittels einer Drossel 16 wird ein Luftstrom über eine Leitung 15 und das Düsenrohr 14 der Düse 12 zugeführt. Durch die Federkraft des Faserbandes wird die Kugel 8 gegen die Düse 12 gedrückt und die Grösse des Luftspaltes zwischen Düse 12 und Kugel 8 stellt sich automatisch so ein, dass der Druck im Rohr zwischen Kugel und Drossel die Presskraft des Faserbandes gerade kompensiert. Dieser Druck ist ein Mass für den Faserquerschnitt. Er kann mittels der Druckmessvorrichtung 17 messbar gemacht und mittels handelsüblicher Wandler in proportionale elektrische Signale umgeformt werden.

Der Luftspalt selbst wird also durch die Grösse der vom Fasermaterial 5 auf die Kugel 8 ausgeübten Pressung bestimmt, so dass der Druck an der Messvorrichtung 17 in einem genau definierten Zusammenhang zur jeweiligen Fasermenge 5 im Kanal 6 steht. Durch die erstmalige Einstellung der Einschraubtiefe des Düsenrohrs 14 im Joch 11 wird lediglich die maximale Grösse des Luftspaltes bestimmt.

Der Vorteil der Anordnung nach Fig. 5 und 6 und ein wesentlicher Teil des erfinderischen Gedankens liegt darin, dass der sphärische Messkörper in einfachster Weise gleichzeitig als Kraftaufnehmer und Druckregler wirkt, und das Messmedium Luft gleichzeitig auch noch als Luftlager und Reinigungsmittel für den Messkörper dient.

Es ist ferner als Vorteil zu betrachten, dass die durch die Düse 12 zugeführte Druckluft nicht oder nur zu einem kleinen Teil an der Kugel 8 vorbei in das Fasermaterial 5 gelangt; der wesentliche Teil entweicht ausserhalb der faserführenden Teile in den Hohlraum 21 und beseitigt dabei allfällige Ablagerungen.

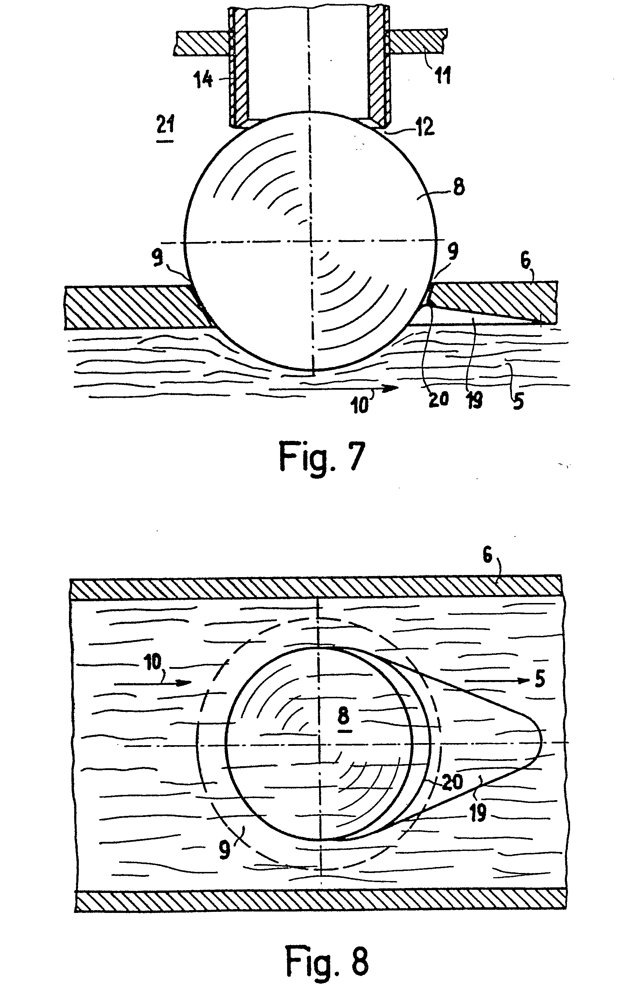

Um zu vermeiden, dass Fasermaterial im Ringspalt zwischen Kanalwand und Kugel 8 festgehalten wird und dadurch zu falschen Messresultaten führt, konnte durch eine in den Fig. 7 und 8 gezeigte Massnahme Abhilfe geschaffen werden. In die konische ringförmige Öffnung 9, in der die Kugel 8 gelagert ist, wird in Richtung des abfliessenden Fasermaterials eine Nute 19 mit polierter Kante 20 eingefräst, deren Achse zur Kanalachse geneigt sein kann. Dadurch werden Fasern, die in den Ringspalt eingedrungen sind, beim Durchlauf an die Hinterseite der Kugel 8 gezogen und werden dort – da der Ringspalt infolge der Nute 19 unterbrochen ist – freigegeben.

Für einen störungsfreien Betrieb der erfindungsgemässen Vorrichtung ist es wesentlich, dass die Kugel 8 auf ihrer ausserhalb des Kanals 6 befindlichen Seite von einem hinreichend grossen Hohlraum 21 umgeben ist, welcher ins Freie führt und durch den die der Düse 12 entströmende Luft unter Mitnahme allfälliger Faser- und Staubpartikel in die Umgebung entweichen kann, so dass ein einwandfreies Funktionieren des Messsystems ohne ständige Wartung garantiert ist.

## Patentansprüche

1. Vorrichtung zur Bestimmung der Substanzmenge oder der Dichte von Faserverbänden mittels eines bezüglich Volumen definierten Raumes mit Fasern oder einem bezüglich Fläche definierten Querschnitt mit Fasern, dadurch gekennzeichnet, dass in dem das Volumen bzw. den Querschnitt definierenden Kanal (6) eine Öffnung (9) vorgesehen ist, in der ein passiver Körper beweglich derart gelagert ist, dass er teilweise in den Kanal (6) hineinragt, welcher Körper durch eine Kraft entgegen der Elastizität der Fasern belastet ist, und dass Mittel zur Messung der Bewegungen des passiven Körpers vorgesehen sind.

2. Vorrichtung nach Anspruch 1, dadurch gekennzeichnet, dass der passive Körper eine Kugel (8) ist, und die Öffnung (9) eine konische Bohrung ist.

3. Vorrichtung nach den Ansprüchen 1 und 2, dadurch gekennzeichnet, dass die auf die Kugel (8) wirkende Kraft durch Druckluft, die mittels einer Düse (12) auf die Kugel gerichtet ist, erzeugt wird, wobei zwischen einer Druckluftquelle (18) und der Düse (12) eine Drossel (16) vorgesehen ist.

4. Vorrichtung nach Anspruch 3, dadurch gekennzeichnet, dass in der Verbindungsleitung (15) zwischen Drossel (16) und Düse (12) eine Druckmessvorrichtung (17) angeschlossen ist.

5. Vorrichtung nach den Ansprüchen 1 und 2, dadurch gekennzeichnet, dass die Wandung des Kanals (6) an der in Durchlaufrichtung der Fasern (5) hinter der Kugel (8) liegenden Zone der Öffnung (9) eine Nut (19) mit polierter Einlaufkante (20) aufweist.

6. Vorrichtung nach den Ansprüchen 1 bis 3, dadurch gekennzeichnet, dass der Teil der Kugel (8), der ausserhalb der Öffnung (9) liegt, sich in einem möglichst grossen Hohlraum (21) befindet, der mit der freien Atmosphäre in Verbindung ist.

## Claims

1. An apparatus for determining the quantity of material or density of fibre structures by means of a space defined with respect to volume and containing fibres or by means of a cross-section defined with respect to its area and containing fibres, characterized in that an opening (9) is provided in the duct (6) defining the volume or cross-section, with a passive body being movably mounted in the opening in such a way that it projects partly into the duct (6), and with the body being loaded by a force against the elasticity of the fibres; and in that means are provided for measuring the movements of the passive body.

2. Apparatus in accordance with Claim 1, characterized in that the passive body is a ball (8) and the opening (9) a conical bore.

3. Apparatus in accordance with Claims 1 and 2, characterized in that the force acting on the ball (8) is generated by compressed air which is directed onto the ball by means of a nozzle (12), with a restrictor (16) being provided between a source of compressed air (18) and the nozzle (12).

4. An apparatus in accordance with Claim 3, characterized in that a pressure measuring device (17) is connected into the connection line (15) between the restrictor (16) and the nozzle (12).

5. Apparatus in accordance with Claims 1 and 2, characterized in that the wall of the duct (6) has a groove (19) with a polished entry edge (20) at the zone of the opening (9) which lies behind the ball (8) in the direction of passage of the fibres (5).

6. Apparatus in accordance with the Claims 1 to 3, characterized in that the part of the ball (8) which lies outside of the opening (9) is located in a hollow cavity which is as large as possible and which communicates with the free atmosphere.

## Revendications

1. Dispositif pour la détermination de la quantité de substance ou de la densité de rubans de fibres au moyen d'un espace contenant des fibres défini en volume ou d'une section transversale contenant des fibres définie en surface, caractérisé en ce qu'il est prévu dans le conduit (6) définissant le volume ou la section transversale une ouverture (9) dans laquelle un corps passif est monté mobile de telle façon qu'il fait saillie en partie à l'intérieur du conduit (6), ce corps étant sollicité par une force s'exerçant à l'encontre de l'élasticité des fibres, et qu'il est prévu des moyens pour mesurer les mouvements du corps passif.

2. Dispositif selon la revendication 1, caractérisé en ce que le corps passif est une boule (8) et que l'ouverture (9) est un trou conique.

3. Dispositif suivant les revendications 1 et 2, caractérisé en ce que la force agissant sur la boule (8) est produite par de l'air comprimé qui est dirigé au moyen d'une buse (12) sur la boule, et en ce qu'il est prévu entre une source d'air comprimé (18) et la buse (12) un papillon d'étranglement (16).

4. Dispositif selon la revendication 3, caractérisé en ce qu'un dispositif manométrique (17) est raccordé dans la conduite de jonction (15) entre le papillon d'étranglement (16) et la buse (12).

5. Dispositif selon les revendications 1 et 2, caractérisé en ce que la paroi du conduit (6) présente dans la zone de l'ouverture (9) située derrière la boule (8) dans le sens de passage des fibres (5) une rainure (19) dont l'arête d'entrée (20) est meulée.

6. Dispositif selon une quelconque des revendications 1 à 3, caractérisé en ce que la partie de la boule (8) qui se trouve à l'extérieur de l'ouverture (9) se trouve dans un espace creux (21) aussi grand que possible qui est en liaison avec l'air libre.

Fig. 1

Fig. 2

Fig. 3

Fig. 4

Fig. 5

Fig. 6

Fig. 7

Fig. 8